# EUROPEAN PATENT APPLICATION

(11) **EP 4 144 734 A1**
(43) Date of publication of application: **08.03.2023**
(21) Application number: 21795639.0
(22) Date of filing: 29.04.2021
(51) Int. Cl.: C07D 471/04, A61P 3/08

(54) **APPLICATION OF HETEROCYCLIC COMPOUND**

(30) Priority: 30.04.2020 CN 202010361015
(71) Applicant: Guangzhou Joyo Pharmatech Co., Ltd, Guangzhou, Guangdong 510730 (CN); Shanghai Jia Tan Pharmatech Co. Ltd., Shanghai 201203 (CN)
(72) Inventor: LI, Yongguo, Guangzhou, Guangdong 510730 (CN); LU, Gang, Shanghai 201203 (CN); LI, Lei, Shanghai 201203 (CN); WEI, Wei, Shanghai 201203 (CN); GE, Zhen, Guangzhou, Guangdong 510730 (CN)
(74) Representative: De Clercq & Partners
(86) International application number: PCT/CN2021/091160
(87) International publication number: WO 2021/219101

(57) **Abstract**

An application of a heterocyclic compound. An application of a substance Y in the preparation of drugs for raising blood sugar. The compound can be used to raise blood sugar and treat hypoglycemia or congenital hyperinsulinism.

## Description

The present application claims the right of the priority of Chinese patent application 202010361015.6 filed on April 30, 2020. The contents of the above Chinese patent application are incorporated herein by reference in its entireties.

### TECHNOLOGY FIELD

The present disclosure relates to use of a heterocyclic compound.

### BACKGROUND

Congenital hyperinsulinemic hypoglycemia (CHI) is a rare endocrine disease that is an important cause of severe and persistent hypoglycemia in newborns and early infants. Under normal physiological conditions, islet β-cells functionally respond to changes in plasma glucose and release insulin, and keeping fasting plasma glucose concentrations within a narrow range of 3.5-5.5 mmol/L. In congenital hyperinsulinemic hypoglycemia, dysregulation of insulin secretion unrelated to glucose metabolism leads to severe and persistent hypoglycemia in newborns and children and a series of severe hypoglycemic brain injuries (Shah et al., Lancet Diabetes Endocrinol 2016).

Recent studies have shown that about 50% of CHI patients can be attributed to mutations in a series of genes associated with dysregulated insulin secretion (ABCC8, KCNJ11, GLUD1, GCK, HADH, SLC16A1, HNF4A, HNF1A, HK1, UCP2, PGM1 and PMM2). Among them, mutations in ABCC8, KCNJ11 account for 40-45% of the total CHI, and mutations in this category respond poorly to the current standard treatment drug diazoxide, accounting for 82% of all diazoxide-null patients (Bellanné-Chantelot C, et al. J Med Genetics 2010;47:752-759.). As the target of diazoxide, ABCC8, KCNJ11 expresses proteins SUR1 and Kir6.2, which are important proteins that constitute the ATP-sensitive potassium channel (K_{ATP}), and mutations in ABCC8, KCNJ11 associated with CHI lead to functional inactivation of K_{ATP}, resulting in excessive insulin secretion and hypoglycemia. (Abu-Osba YK et al., Arch Dis Child 1989; 64(10): 1496-1500; Gary KD et al., J Perinatol. 2018; 38(11): 1496-1502.). In addition to genetic factors, pathological studies have found that CHI is accompanied by pathological changes in the pancreatic tissue, which are generally divided into two main forms of proliferation: morphologically focal (Focal disease) and diffuse (Diffuse disease).

Current treatment for CHI includes drug and surgical treatment, as well as corresponding dietary control. The goal of treatment is to maintain blood glucose above 3.5 mmol/L. Diazoxide is a primary drug for long-term glycemic control, and its main mechanism is to act as an ATP-sensitive potassium channel (K_{ATP}) opener, causing hyperpolarization of islet β-cells and thus reducing insulin secretion. However, long-term use of diazoxide produces a variety of side effects, including hirsutism, edema, hyperuricemia, tachycardia, hypertrophy, leukopenia, intolerance, pulmonary hypertension, severe hypotension, and heart failure. Also some CHI patients with site-specific mutations (e.g., ABCC8, KCNJ11) do not respond well to diazoxide. In addition to diazoxide, somatostatin analogues such as octreotide and glucagon have also been tried for the treatment of CHI patients. Partial or near-complete surgical resection of the pancreas becomes the only option after diazerazine and other drugs are ineffective, whereas such surgery is limited to patients with focal CHI. For diffuse CHI, even near-complete resection of the pancreas cannot completely relieve symptoms, and additional sequelae of surgery including decreased quality of life and diabetes due to insufficient insulin secretion are a major drawback of treatment (Shah et al., Lancet Diabetes Endocrinol 2016). Therefore, there remains a widespread and urgent medical need for the treatment of CHI.

In addition, hypoglycemia can lead to a variety of symptoms, including lack of coordination, confusion of consciousness, loss of consciousness, seizures and even death. Most episodes of mild hypoglycemia can be effectively self-treated by ingesting glucose tablets or other beverages or snacks containing carbohydrates. More severe symptomatic hypoglycemia can also be treated by oral intake of carbohydrates. However, when a hypoglycemic patient is unable to take oral glucose supplements due to confusion of consciousness, delirium, or other reasons, parenteral therapy is required. As a non-hospital rescue procedure, the hyperglycemic hormone-glucagon is sometimes administered subcutaneously or intramuscularly by the patient himself or by a fellow patient trained to recognize and treat severe hypoglycemia.

Novartis' Alpelisib (BYL719) has obvious gastrointestinal side effects in the Phase III clinical trial (N=287), with a diarrhea incidence of 57.7% (6.7% above grade 3), a nausea incidence of 44.7% (2.5% above grade 3), and a vomiting incidence of 27.1% (0.7% above grade 3) (reference: Fabrice André et al., N Engl J Med 2019; 380: 1929-40).

### CONTENT OF THE PRESENT INVENTION

The technical problem to be solved by the present disclosure is that the existing drugs for raising blood glucose, treating hypoglycemia or treating congenital hyperinsulinism have a relatively simple structure, poor clinical therapeutic treatment effect and high side effects, etc. To this end, the present disclosure provides use of a heterocyclic compound that can be used to raise blood glucose, treat hypoglycemia or congenital hyperinsulinism.

The present disclosure provides use of a substance **Y** or a pharmaceutical composition thereof in the manufacture of a medicament for one or more of raising blood glucose, treating hypoglycemia and treating congenital hyperinsulinism; the pharmaceutical composition consists of the substance **Y** and at least one pharmaceutical excipient;

the substance **Y** is a substance **B**, a pharmaceutically acceptable salt thereof, a solvate thereof, or a solvate of the pharmaceutically acceptable salt thereof; the substance **B** is

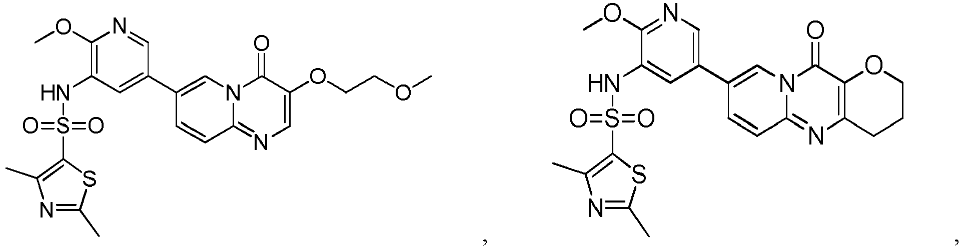

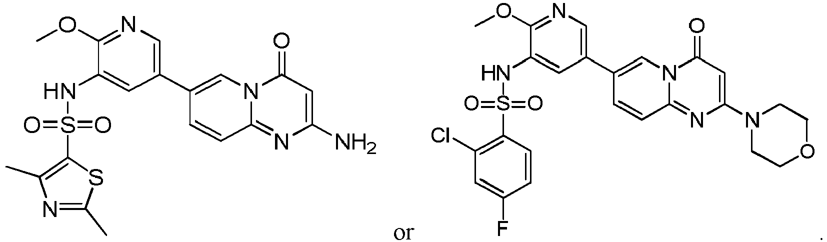

In the use of the substance **Y** or the pharmaceutical composition thereof in the manufacture of the medicament for one or more of raising blood glucose, treating hypoglycemia and treating congenital hyperinsulinism, the pharmaceutical composition can consist of the following components in mass fraction: 0.5% of substance **B**, 20.0% of microcrystalline cellulose, 72.0% of mannitol, 4.0% of cross-linked sodium carboxymethylcellulose, 3.0% of hydroxypropyl methyl cellulose, 0.5% magnesium stearate; preferably, the mass of the substance **B** is 0.1-1.4 mg.

In the use of the substance **Y** or the pharmaceutical composition thereof in the manufacture of the medicament for one or more of raising blood glucose, treating hypoglycemia and treating congenital hyperinsulinism, the medicament can be presented in a tablet form.

In the use of the substance **Y** or the pharmaceutical composition thereof in the manufacture of the medicament for one or more of raising blood glucose, treating hypoglycemia and treating congenital hyperinsulinism, the medicament can be administered orally.

In the use of the substance **Y** or the pharmaceutical composition thereof in the manufacture of the medicament for one or more of raising blood glucose, treating hypoglycemia and treating congenital hyperinsulinism, a single administration dose of the medicament can be 0.1-1.4 mg, such as 0.1 mg, 0.2 mg, 0.4 mg, 0.5 mg, 0.7 mg, 0.9 mg, 1.1 mg or 1.4 mg.

In the use of the substance **Y** or the pharmaceutical composition thereof in the manufacture of the medicament for one or more of raising blood glucose, treating hypoglycemia and treating congenital hyperinsulinism, an administration frequency of the medicament can be 1 time/day, 2 times/day or 3 times/day.

In the use of the substance **Y** or the pharmaceutical composition thereof in the manufacture of the medicament for one or more of raising blood glucose, treating hypoglycemia and treating congenital hyperinsulinism, an administration dose of the medicament can be 0.1-1.4 mg/day, such as 0.1 mg/day, 0.2 mg/day, 0.4 mg/day, 0.5 mg/day, 0.7 mg/day, 0.9 mg/day, 1.1 mg/day or 1.4 mg/day.

The present disclosure also provides a substance **Y** or a pharmaceutical composition thereof for use in one or more of raising blood glucose, treating hypoglycemia and treating congenital hyperinsulinism; the pharmaceutical composition consists of the substance **Y** and at least one pharmaceutical excipient;

the substance **Y** is a substance **B**, a pharmaceutically acceptable salt thereof, a solvate thereof, or a solvate of the pharmaceutically acceptable salt thereof; the substance **B** is

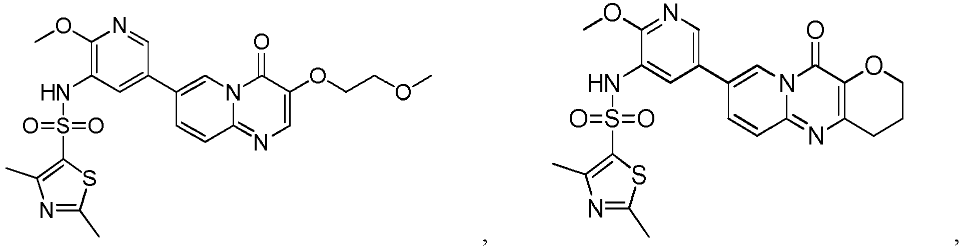

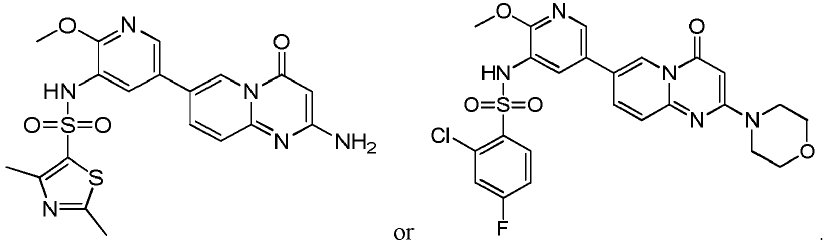

In the substance **Y** or the pharmaceutical composition thereof for use in one or more of raising blood glucose, treating hypoglycemia and treating congenital hyperinsulinism, the pharmaceutical composition can consist of the following components in mass fractions: 0.5% of substance **B**, 20.0% of microcrystalline cellulose, 72.0% of mannitol, 4.0% of cross-linked sodium carboxymethylcellulose, 3.0% of hydroxypropyl methyl cellulose, and 0.5% of magnesium stearate; preferably, the mass of the substance **B** is 0.1-1.4 mg.

The present disclosure also provides a method for one or more of raising blood glucose, treating hypoglycemia and treating congenital hyperinsulinism, comprising administering to a patient a therapeutically effective amount of a substance **Y** or a pharmaceutical composition thereof; the pharmaceutical composition consists of the substance **Y** and at least one pharmaceutical excipient;

the substance **Y** is a substance **B**, a pharmaceutically acceptable salt thereof, a solvate thereof, or a solvate of the pharmaceutically acceptable salt thereof; the substance **B** is

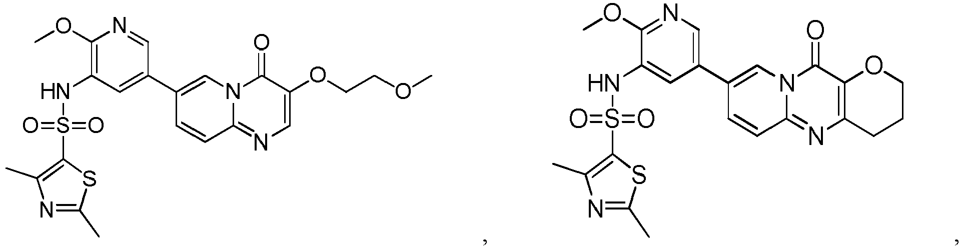

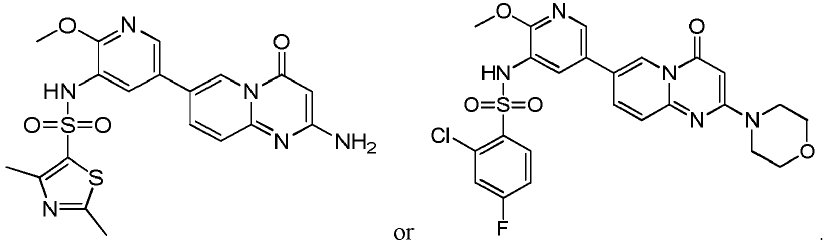

In the method for one or more of raising blood glucose, treating hypoglycemia and treating congenital hyperinsulinism, the pharmaceutical composition can consist of the following components in mass fractions: 0.5% of substance **B**, 20.0% of microcrystalline cellulose, 72.0% of mannitol, 4.0% of cross-linked sodium carboxymethylcellulose, 3.0% of hydroxypropyl methyl cellulose, 0.5% of magnesium stearate; preferably, the mass of the substance **B** is 0.1-1.4 mg.

The present disclosure also provides a pharmaceutical composition 1 or a pharmaceutical composition 2, wherein

the pharmaceutical composition 1 consists of the following components in mass fractions: 0.5% of substance **Y**, 20.0% of microcrystalline cellulose, 72.0% of mannitol, 4.0% of cross-linked sodium carboxymethylcellulose, 3.0% of hydroxypropyl methyl cellulose, 0.5% of magnesium stearate;

the pharmaceutical composition 2 consists of a substance **Y** and at least one pharmaceutical excipient;

in the pharmaceutical composition 1 and pharmaceutical composition 2, the substance **Y** is a substance **B**, a pharmaceutically acceptable salt thereof, a solvate thereof, or a solvate of the pharmaceutically acceptable salt thereof; the substance **B** is 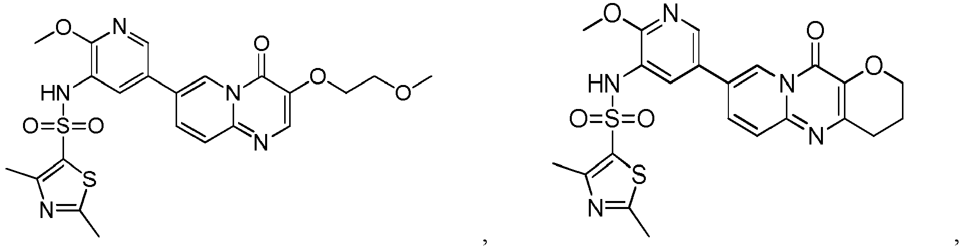 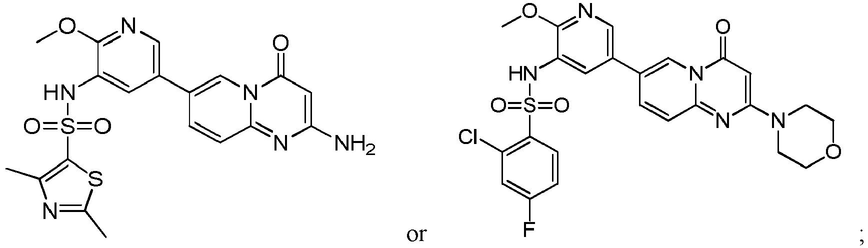 in the pharmaceutical composition 2, the mass of the substance **B** in the substance **Y** is 0.1 to 1.4 mg.

In a certain embodiment, the mass of the substance **B** in the pharmaceutical composition 1 can be 0.1-1.4 mg.

In a certain embodiment, the pharmaceutical composition 1 or pharmaceutical composition 2 can be presented in a tablet form.

Unless otherwise specified, the terms used in the present disclosure have the following meanings:

The term "pharmaceutically acceptable" refers to salts, solvents, excipients and the like that are generally non-toxic, safe, and suitable for patient use. The "patient" is preferably a mammal, more preferably a human being.

The term "pharmaceutically acceptable salt" refers to salt prepared from the compound of the present disclosure and a relatively non-toxic and pharmaceutically acceptable acid or alkali. When the compound of the present disclosure contains relatively acidic functional groups, an alkali addition salt can be obtained by allowing a sufficient amount of pharmaceutically acceptable alkali to be in contact with the prototype of the compound in a pure solution or an appropriate inert solvent. The pharmaceutically acceptable alkali addition salt includes, but is not limited to, lithium salt, sodium salt, potassium salt, calcium salt, aluminium salt, magnesium salt, zinc salt, bismuth salt, ammonium salt, and diethanolamine salt. When the compound of the present disclosure contains relatively alkaline functional groups, an acid addition salt can be obtained by allowing a sufficient amount of pharmaceutically acceptable acid to be in contact with the prototype of the compound in a pure solution or an appropriate inert solvent. The pharmaceutically acceptable acid includes inorganic acid, and the inorganic acid includes, but is not limited to, hydrochloric acid, hydrobromic acid, hydroiodic acid, nitric acid, carbonic acid, phosphoric acid, phosphorous acid, sulfuric acid, etc. The pharmaceutically acceptable acid includes organic acid, and the organic acid includes, but is not limited to, acetic acid, propionic acid, oxalic acid, isobutyric acid, maleic acid, malonic acid, benzoic acid, succinic acid, suberic acid, *trans*-butenedioic acid, lactic acid, mandelic acid, phthalic acid, benzenesulfonic acid, *p-*toluenesulfonic acid, citric acid, salicylic acid, tartaric acid, methanesulfonic acid, isonicotinic acid, acidic citric acid, oleic acid, tannic acid, pantothenic acid, bitartrate, ascorbic acid, gentisic acid, fumaric acid, gluconic acid, saccharic acid, formic acid, ethanesulfonic acid, pamoic acid (i.e., 4,4'-methylene-bis(3-hydroxy-2-naphthoic acid)), amino acid (e.g., glutamic acid and arginine), etc. When the compound of the present disclosure contains relatively acidic functional groups and relatively alkaline functional groups, it can be converted into an alkali addition salt or an acid addition salt. For details, see Berge et al., "Pharmaceutical Salts", Journal of Pharmaceutical Science 66: 1-19 (1977), or Handbook of Pharmaceutical Salts: Properties, Selection, and Use (P. Heinrich Stahl and Camille G. Wermuth, ed., Wiley-VCH, 2002).

The term "solvate" refers to a substance formed by combining the compound of the present disclosure with a stoichiometric or non-stoichiometric amount of solvent. Solvent molecules in the solvate can exist in an ordered or non-ordered arrangements. The solvent includes, but is not limited to, water, methanol, ethanol, etc.

In the term "solvate of pharmaceutically acceptable salt", the "pharmaceutically acceptable salt" and the "solvate" are as described above, and the "solvate of the pharmaceutically acceptable salt" refers to a substance formed by combining the compound of the present disclosure, a relatively non-toxic and pharmaceutically acceptable acid or alkali of 1 with a stoichiometric or non-stoichiometric amount of solvent of 2. The "solvate of pharmaceutically acceptable salt" includes, but is not limited to, a hydrochloride monohydrate of the compound of the present disclosure.

The term "treatment" refers to a therapeutic therapy. Regarding to a specific disease, the treatment refers to: (1) alleviation of one or more biological manifestations of a disorder or disease, (2) interfering with (a) one or more points in the biological cascade leading to or causing a disease or (b) one or more biological manifestations of the disease, (3) improvement of one or more symptoms, effects or side effects associated with the disease, or one or more symptoms, effects or side effects associated with the disease or treatment thereof, or (4) slowdown of the progression of a disease or one or more biological manifestations of the disease.

The term "prevention" refers to a reduced risk of acquiring or developing a disease or disorder.

The term "therapeutically effective amount" refers to an amount of compound that, when administered to a patient, is sufficient to effectively treat the disorders or diseases described herein. The "therapeutically effective amount" is changed according to the compound, the disease and its severity, and the age of the patient to be treated, but can be adjusted as needed by those skilled in the art.

The term "patient" refers to any animal, preferably a mammal, and most preferably a human, to whom the compound will be or has been administered according to embodiments of the present disclosure. The term "mammal" includes any mammal. Examples of the mammal include, but are not limited to, cow, horse, sheep, pig, cat, dog, mouse, rat, rabbit, guinea pig, monkey, human, etc., and most preferably a human.

The term "pharmaceutical excipient" refers to the vehiculum and additive used in the manufacture of a medicament and the formulation of prescriptions, which are all substances contained in pharmaceutical preparations except active ingredients. See Pharmacopoeia of the People's Republic of China (2020 Edition), or, Handbook of Pharmaceutical Excipients (Raymond C Rowe, 2009).

The above preferred conditions can be combined arbitrarily to obtain preferred embodiments of the present disclosure without violating common knowledge in the art.

All reagents and raw materials used in the present disclosure are commercially available.

The positive and progressive effect of the present disclosure is that the use of the heterocyclic compounds of the present disclosure, the compounds can be used to raise blood glucose, treat hypoglycemia or treat congenital hyperinsulinism.

### DETAILED DESCRIPTION OF THE PREFERRED EMBODIMENT

The present disclosure will be further described below with reference to embodiments, but the present disclosure is not therefore limited to the scope of the embodiment. Experimental methods without specific conditions in the following embodiments are selected according to conventional methods and conditions, or according to the commercial specification.

The embodiments were all derived from a clinical trial study. The subjects were screened and eligible to enter groups according to the entry criteria specified in the clinical protocol. The test drug was administered orally on day 1, and adverse events and combined administration were observed and recorded after administration. After safety evaluation was conducted on day 7 of the single administration period, the subjects received continuous administration of the test drug until the subjects withdrew from the clinical trial. Safety assessments were conducted during the clinical trial according to the time specified in the clinical protocol.

Compound 11A in the following embodiments refers to 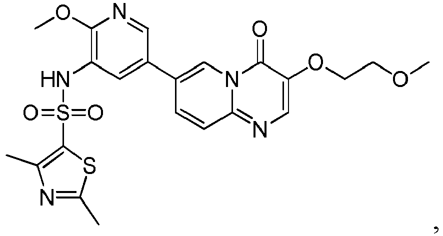 supplied by Shanghai Jiatan Pharmatech Co., LTD., prepared according to CN 105461712A, embodiment 176.

### Embodiment 1

In the present disclosure, the drugs can be presented in a tablet form, and the mass fractions of each component contained in the tablet are as shown in the following table 1:

**Table 1**

| **Percentage of raw auxiliary materials** | **(w/w) %** |
|---|---|
| 11A | 0.5 |
| Microcrystalline cellulose | 20.0 |
| Mannitol | 72.0 |
| Cross-linked sodium carboxymethylcellulose | 4.0 |
| Hydroxypropyl methyl cellulose | 3.0 |
| Magnesium stearate | 0.5 |
| Total | 100.0 |

.

The preparation method of the tablets:
1. The prescription amount of substance 11A was weighed, and 4 times the amount of mannitol was added, and the mixture was repeatedly passed through the 40-mesh sieve to mix evenly;
2. an additional 4 times the amount of mannitol was added to the mixture from the step 1, and the mixture was mixed in a suitable mixing drum at 20 rpm for 15 minutes;
3. the previous operation in step 2 was repeated until the amount of mixture reached 10% or more of the total mixture, and then the remaining materials other than magnesium stearate were added thereto to mix at 20 rpm for 15 minutes;
4. the prescription amount of magnesium stearate was added to the mixture in step 3 to mix at 20 rpm for 3 minutes;
5. the above mixture was sampled for mixing uniformity, and after qualification, the mixture was pressed and packaged according to the theoretical sheet weight, so as to obtain the product.

### Embodiment 2

Case data: Subject 1, male, 56 years old, signed the informed consent on October 25, 2018. During the baseline period, vital signs, physical examination, blood routine, blood biochemical, coagulation function, urine routine and other examinations were performed, wherein the fasting blood glucose value was 5.64 mmol/L. The tablet containing 0.1 mg of compound 11A was administered for single administration on November 2, 2018. One week later, the tablet containing 0.1 mg of compound 11A was administered for continuous administration once a day on November 9, 2018. The case was followed up for blood routine, blood biochemical and other examinations on March 14, 2019, and the fasting blood glucose increased to 6.19 mmol/L.

### Embodiment 3

Case data: Subject 2, male, 64 years old, signed the informed consent on January 04, 2019. During the baseline period, vital signs, physical examination, blood routine, blood biochemical, coagulation function, urine routine and other examinations were performed, wherein the fasting blood glucose value was 5.81 mmol/L. The tablet containing 0.2 mg of compound 11A was administered for single administration on January 9, 2019. One week later, the tablet containing 0.2 mg of compound 11A was administered for continuous administration once a day on January 16, 2019. The case was followed up for blood routine, blood biochemical and other examinations on January 23, 2019, and the fasting blood glucose increased to 6.45 mmol/L.

### Embodiment 4

Case data: Subject 3, male, 37 years old, signed the informed consent on May 13, 2019. During the baseline period, vital signs, physical examination, blood routine, blood biochemical, coagulation function, urine routine and other examinations were performed, wherein the fasting blood glucose value was 5.63 mmol/L. The tablet containing 0.4 mg of compound 11A was administered for single administration on May 23, 2019. One week later, the tablet containing 0.4 mg of compound 11A was administered for continuous administration once a day on May 30, 2019. The case was followed up for blood routine, blood biochemical and other examinations on June 5, 2019, and the fasting blood glucose increased to 6.99 mmol/L.

### Embodiment 5

Case data: Subject 5, male, 56 years old, signed the informed consent on October 22, 2019. During the baseline period, vital signs, physical examination, blood routine, blood biochemical, coagulation function, urine routine and other examinations were performed, wherein the fasting blood glucose value was 6.06 mmol/L. The tablet containing 0.7 mg of compound 11A was administered for single administration on October 31, 2019. One week later, the tablet containing 0.7 mg of compound 11A was administered for continuous administration once a day on November 7, 2019. The case was followed up for blood routine, blood biochemical and other examinations on November 13, 2019, and the fasting blood glucose increased to 8.37 mmol/L. After continuous administration once a day, the case was followed up on November 20, 2019, and fasting blood glucose increased to 9.44 mmol/L.

### Embodiment 6

Case data: Subject 6, male, 52 years old, signed the informed consent on November 05, 2019. During the baseline period, vital signs, physical examination, blood routine, blood biochemical, coagulation function, urine routine and other examinations were performed, wherein the fasting blood glucose value was 5.87 mmol/L. The tablet containing 0.7 mg of compound 11A was administered for single administration on November 14, 2019. One week later, the tablet containing 0.7 mg of compound 11A was administered for continuous administration once a day on November 21, 2019. The case was followed up for blood routine, blood biochemical and other examinations on November 27, 2019, and the fasting blood glucose increased to 11.4 mmol/L. After stopping administering tablets containing 0.7 mg of compound 11A on November 27, 2019, the fasting blood glucose returned to normal on November 29, 2019. The tablet containing 0.7 mg of compound 11A was administered continuously on November 30, 2019. The case was followed up on December 4, 2019, and the fasting blood glucose increased to 11.44 mmol/L.

### Embodiment 7

Case data: subject 7, female, 50 years old, signed informed consent on November 26, 2019. During the baseline period, vital signs, physical examination, blood routine, blood biochemical, coagulation function, urine routine and other examinations were performed, and the fasting blood glucose value was 5.92 mmol/L. The tablet containing 0.7 mg of compound 11A was administered for single administration on December 3, 2019. One week later, the tablet containing 0.7 mg of compound 11A was administered for continuous administration once a day on December 10, 2019. The case was followed up for blood routine, blood biochemical and other examinations on January 20, 2020, and the fasting blood glucose increased to 9.13 mmol/L.

### Embodiment 8

Case data: Subject 8, female, 48 years old, signed the informed consent on December 3, 2020. During the baseline period, vital signs, physical examination, blood routine, blood biochemical, coagulation function, urine routine and other examinations were performed, wherein the fasting blood glucose value was 6.87 mmol/L. The tablet containing 1.1 mg of compound 11A was administered for single administration on December 10, 2020. One week later, the tablet containing 1.1 mg of compound 11A was administered for continuous administration once a day on December 17, 2019. The fasting blood glucose increased to 12.66 mmol/L on December 23, 2020.

### Embodiment 9

Case data: Subject 9, female, 28 years old, signed the informed consent on August 6, 2020. During the baseline period, vital signs, physical examination, blood routine, blood biochemical, coagulation function, urine routine and other examinations were performed, wherein the fasting blood glucose value was 5.94 mmol/L. The tablet containing 1.4 mg of compound 11A was administered for single administration on August 20, 2020. One week later, the tablet containing 1.4 mg of compound 11A was administered for continuous administration once a day on August 27, 2020. The fasting blood glucose increased to 10.77 mmol/L on September 2, 2020.

Embodiments 2 to 9 were derived from an ongoing clinical trial study with dose groups of 0.1 mg, 0.2 mg, 0.4 mg, 0.5 mg, 0.7 mg, 0.9 mg, 1.1 mg, and 1.4 mg. The incidence of hyperglycemia in these subjects was 80% during the administering period, which shows that compound 11A is very effective in raising blood glucose. In addition, there were no significant gastrointestinal side effects such as nausea, vomiting and diarrhea in these 8 subjects of above embodiments.

The clinical trial research method of any of the following compounds was the same as that of compound 11A as described above. 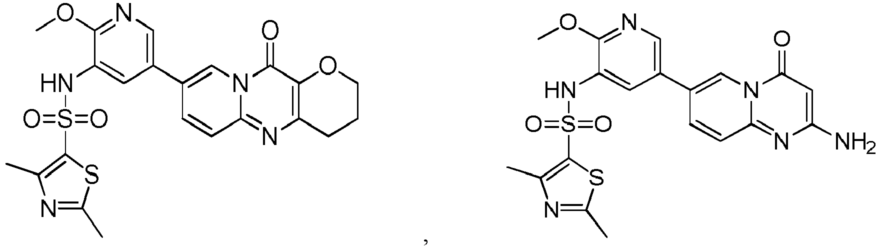 or

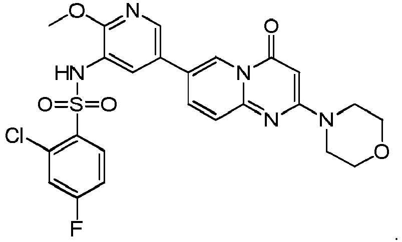

### Embodiment 10

### Bioavailability experiments

### 1. Experimental animals

The 12 non-naive beagle dogs (6/sex) in this experiment were provided by Beijing Marshall Biotechnology Co., Ltd. Each animal had a tattoo number on its ear as an identification code. During the experimental period, animals were housed in single cages with animal identification codes marked on the cage tags. The 12 non-naive beagle dogs (6/sex) were randomly divided into 2 groups (3/sex/group).

### 2. Experimental design

(1) Non-naive beagle dogs were administered intravenously (solvent 50% PEG400 + 50% PBS) at a dose volume of 2 mL/kg per dog (dose of 0.1 mg/kg per dog), and then blood was collected to measure the plasma drug concentration of non-naive beagle dogs at different time points of 15 minutes, 30 minutes, 1 hour, 2 hours, 4 hours, 6 hours, 8 hours, and 24 hours.
(2) Non-naive beagle dogs were administered orally at a dose volume of 1 tablet per dog (dose of 0.5 mg per dog), and then blood was collected to measure the plasma drug concentration of non-naive beagle dogs at different time points of 15 minutes, 30 minutes, 1 hour, 2 hours, 4 hours, 6 hours, 8 hours, and 24 hours; the oral bioavailability of tablets was calculated.

### 3. Pharmacokinetic data analysis

Plasma drug concentration data was processed in a non-compartment model using WinNonlin^{™} Version 6.2.1 (Pharsight, Mountain View, CA) pharmacokinetic software. The peak concentration (Cₘₐₓ), peak time (Tₘₐₓ) and quantifiable time were directly read out from the plasma drug concentration-time diagram.

The following pharmacokinetic parameters were calculated using the log-linear trapezoidal method: elimination phase half-life (T_{1/2}), apparent volume of distribution (Vdₛₛ) and clearance rate (CL), mean residence time of the drug from 0 to the end time point *in vivo* (MRT₀₋ₗₐₛₜ), mean residence time of the drug from 0 to infinite time *in vivo* (MRT_{0-inf}), the area under the time-plasma concentration curve from 0 to the end time point (AUC₀₋ₗₐₛₜ), the area under the time-plasma concentration curve from 0 to infinite time (AUC_{0-inf}), initial concentration (C₀).

### 4. Experimental conclusion

After a single intravenous injection of 11A of 0.1 mg/kg, male and female beagle dogs showed a moderately high plasma clearance rate (CL) of 24.6±6.66 mL/min/kg, and the apparent volume of distribution (Vdₛₛ) of 6.16±1.91 L/kg showed that the compound was widely distributed *in vivo.* The mean plasma elimination phase half-life T_{1/2} and area under the curve AUC₀₋ₗₐₛₜ were 3.21±1.81 h and 114±27.6 nM*h, respectively.

After a single oral administration of tablets containing 0.5 mg of 11A prepared from the prescription of embodiment 1 in male and female beagle dogs, the bioavailability was 80.9% and the peak concentration (Cₘₐₓ) was 11.1±2.08, and the peak times occurred at 1.00±0.00 h after administration, respectively.

## Claims

1. Use of a substance **Y** or a pharmaceutical composition thereof in the manufacture of a medicament for one or more of raising blood glucose, treating hypoglycemia and treating congenital hyperinsulinism; the pharmaceutical composition consists of the substance **Y** and at least one pharmaceutical excipient;
the substance **Y** is a substance **B**, a pharmaceutically acceptable salt thereof, a solvate thereof, or a solvate of the pharmaceutically acceptable salt thereof; the substance **B** is

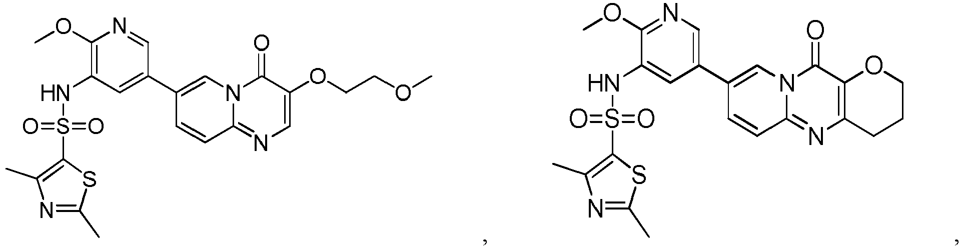

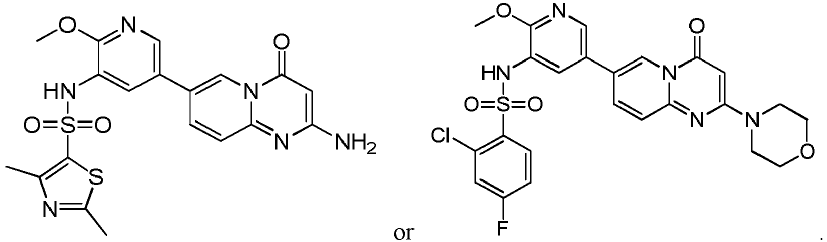

2. The use of the substance **Y** or the pharmaceutical composition thereof in the manufacture of the medicament for one or more of raising blood glucose, treating hypoglycemia and treating congenital hyperinsulinism as claimed in claim 1, wherein the use satisfies one or more of the following conditions:
(1) the pharmaceutical composition consists of the following components in mass fractions: 0.5% of substance **B**, 20.0% of microcrystalline cellulose, 72.0% of mannitol, 4.0% of cross-linked sodium carboxymethylcellulose, 3.0% of hydroxypropyl methyl cellulose, 0.5% of magnesium stearate;
(2) the medicament is presented in a tablet form;
(3) the medicament is administered orally;
(4) a single administration dose of the medicament is 0.1-1.4 mg, such as 0.1 mg, 0.2 mg, 0.4 mg, 0.5 mg, 0.7 mg, 0.9 mg, 1.1 mg or 1.4 mg;
(5) an administration frequency of the medicament is 1 time/day, 2 times/day or 3 times/day;
(6) an administration dose of the medicament is 0.1-1.4 mg/day, such as 0.1 mg/day, 0.2 mg/day, 0.4 mg/day, 0.5 mg/day, 0.7 mg/day, 1.1 mg/day, or 1.4 mg/day.

3. The use of the substance **Y** or the pharmaceutical composition thereof in the manufacture of the medicament for one or more of raising blood glucose, treating hypoglycemia and treating congenital hyperinsulinism as claimed in claim 1, wherein the mass of the substance **B** is 0.1-1.4 mg.

4. A substance **Y** or a pharmaceutical composition thereof for use in one or more of raising blood glucose, treating hypoglycemia and treating congenital hyperinsulinism; the pharmaceutical composition consists of the substance **Y** and at least one pharmaceutical excipient;
the substance **Y** is a substance **B**, a pharmaceutically acceptable salt thereof, a solvate thereof, or a solvate of the pharmaceutically acceptable salt thereof; the substance **B** is

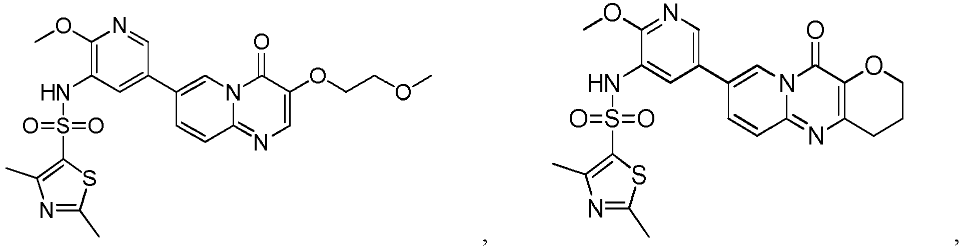

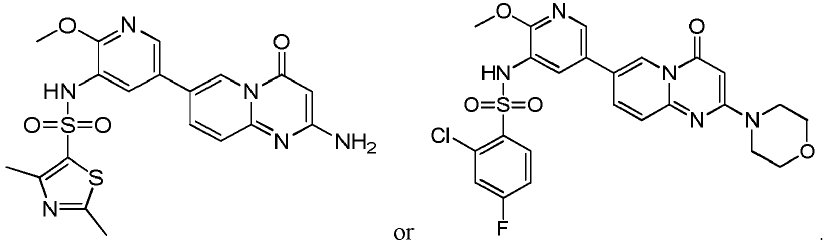

5. The substance **Y** or the pharmaceutical composition thereof for use in one or more of raising blood glucose, treating hypoglycemia and treating congenital hyperinsulinism as claimed in claim 4, wherein the pharmaceutical composition consists of the following components in mass fraction: 0.5% of substance **B**, 20.0% of microcrystalline cellulose, 72.0% of mannitol, 4.0% of cross-linked sodium carboxymethylcellulose, 3.0% of hydroxypropyl methyl cellulose, 0.5% of magnesium stearate; preferably, the mass of the substance **B** is 0.1-1.4 mg.

6. A method for one or more of raising blood glucose, treating hypoglycemia and treating congenital hyperinsulinism, comprising administering to a patient a therapeutically effective amount of a substance **Y** or a pharmaceutical composition thereof; the pharmaceutical composition consists of the substance **Y** and at least one pharmaceutical excipient;
the substance **Y** is a substance **B**, a pharmaceutically acceptable salt thereof, a solvate thereof, or a solvate of the pharmaceutically acceptable salt thereof; the substance **B** is

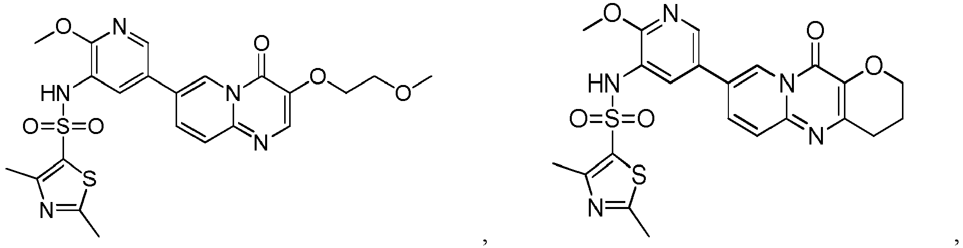

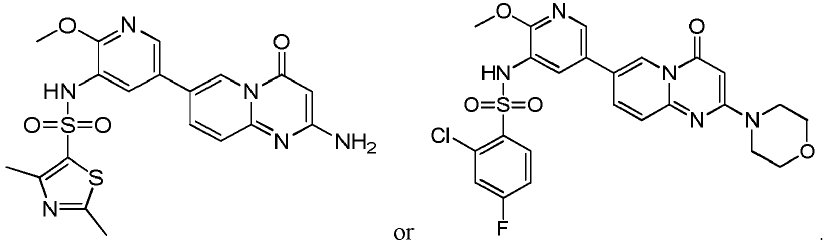

7. The method for one or more of raising blood glucose, treating hypoglycemia and treating congenital hyperinsulinism as claimed in claim 6, wherein the pharmaceutical composition consists of the following components in mass fraction: 0.5% of substance **B**, 20.0% of microcrystalline cellulose, 72.0% of mannitol, 4.0% of cross-linked sodium carboxymethylcellulose, 3.0% of hydroxypropyl methyl cellulose, 0.5% of magnesium stearate; preferably, the mass of the substance **B** is 0.1-1.4 mg.

8. A pharmaceutical composition 1 or a pharmaceutical composition 2, wherein,
the pharmaceutical composition 1 consists of the following components in mass fractions: 0.5% of substance **Y**, 20.0% of microcrystalline cellulose, 72.0% of mannitol, 4.0% of cross-linked sodium carboxymethylcellulose, 3.0% of hydroxypropyl methyl cellulose, 0.5% of magnesium stearate;
the pharmaceutical composition 2 consists of a substance **Y** and at least one pharmaceutical excipient;
in the pharmaceutical composition 1 and pharmaceutical composition 2, the substance **Y** is a substance **B**, a pharmaceutically acceptable salt thereof, a solvate thereof, or a solvate of the pharmaceutically acceptable salt thereof; the substance **B** is 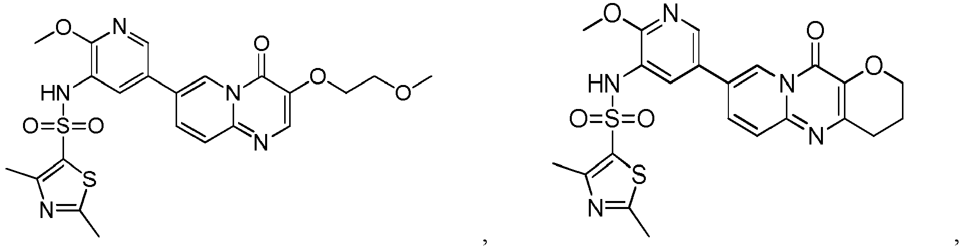 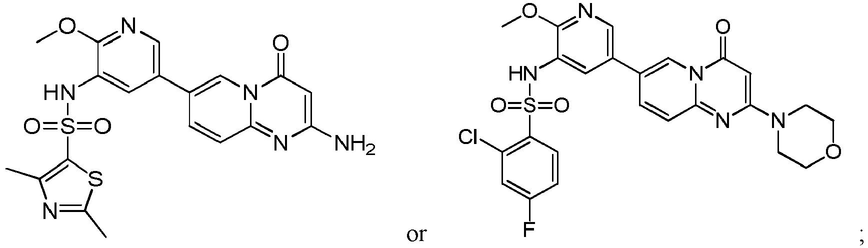 in the pharmaceutical composition 2, the mass of the substance **B** in the substance **Y** is 0.1 to 1.4 mg.

9. The pharmaceutical composition 1 or pharmaceutical composition 2 as claimed in claim 8, wherein the pharmaceutical composition 1 or pharmaceutical composition 2 is presented in a tablet form.

10. The pharmaceutical composition 1 or pharmaceutical composition 2 as claimed in claim 8, wherein the mass of the substance **B** in the pharmaceutical composition 1 is 0.1-1.4 mg.
